# FASCICULE DE BREVET EUROPEEN

(11) **EP 2 895 435 B1**
(45) Date de publication et mention de la délivrance du brevet: **09.12.2020**
(21) Numéro de dépôt: 13759469.3
(22) Date de dépôt: 02.09.2013
(51) Int. Cl.: C03C 17/38, C23C 18/16, C23C 18/18, G02B 5/08

(54) **PROCEDE DE FABRICATION D'UN MIROIR SANS COUCHE DE CUIVRE**
VERFAHREN ZUR HERSTELLUNG EINES SPIEGELS OHNE KUPFERSCHICHT
METHOD FOR MANUFACTURING A MIRROR WITH NO COPPER LAYER

(30) Priorité: 17.09.2012 BE 201200612
(43) Date de publication de la demande: 22.07.2015
(73) Titulaire: AGC Glass Europe, 1348 Louvain-La-Neuve (BE)
(72) Inventeur: PIETERS, Ronny, 1470 Bousval/Genappe (BE); ATTOUT, Anne, B-6220 Fleurus (BE)
(74) Mandataire: AGC Glass Europe
(86) Numéro de dépôt international: PCT/EP2013/068054
(87) Numéro de publication internationale: WO 2014/040870

(56) Documents cités:
- WO-A1-2010/037867
- FR-A1- 2 641 271
- US-A- 6 017 580
- US-A1- 2011 261 473

## Description

### 1. Domaine de l'invention

L'invention concerne des miroirs et des procédés de fabrication de tels miroirs.

Les miroirs selon cette invention peuvent avoir diverses applications, par exemple: miroirs domestiques utilisés entre autre dans des meubles, garde-robes ou salles-de-bain; miroirs pour boîtes de maquillage ou poudriers; miroirs utilisés dans l'industrie automobile, comme rétroviseurs de véhicules par exemple. Cette invention peut être également avantageuse pour des miroirs utilisés comme réflecteurs d'énergie solaire.

### 2. Solutions de l'art antérieur

Les miroirs domestiques et miroirs pour applications solaires ont généralement été produits ainsi: une feuille de verre plat (verre flotté, sodo-calcique) est d'abord polie et rincée, puis, sensibilisée au moyen d'une solution de chlorure d'étain; après rinçage, la surface du verre est traditionnellement activée par traitement avec une solution ammoniacale de nitrate d'argent, ensuite une solution d'argenture est appliquée de manière à former une couche d'argent; cette couche d'argent est ensuite recouverte d'une couche protectrice de cuivre. Après séchage, une ou plusieurs couches de peinture contenant du plomb sont déposées afin de produire le miroir fini. Le but de la couche de cuivre est de retarder le ternissement de la couche d'argent, et la couche de cuivre est elle-même protégée de l'abrasion et de la corrosion par la couche de peinture. Des formulations différentes de peintures peuvent être utilisées pour protéger un miroir. Cependant, celles qui offrent la meilleure protection contre la corrosion de la couche de cuivre contiennent du plomb. Malheureusement ce plomb est toxique et son utilisation est de plus en plus déconseillée pour des raisons de protection de la santé et de l'environnement.

Glaverbel a notamment développé des miroirs sans la couche de cuivre conventionnellement utilisée, qui permettent l'utilisation de peintures essentiellement sans plomb tout en conservant les propriétés du miroir et une résistance à la corrosion et au vieillissement acceptable ou même améliorée. Par exemple, le brevet US 6,565, 217 décrit un miroir sans couche de cuivre qui comprend dans l'ordre: un substrat en verre; de l'étain et au moins un matériel choisi dans le groupe du palladium, du bismuth, du chrome, de l'or, de l'indium, du nickel, du platine, du rhodium, du ruthénium, du titane, du vanadium et du zinc déposés sur une surface du substrat en verre; une couche de revêtement d'argent sur ladite surface du substrat; au moins un matériel choisi dans le groupe de l'étain, du chrome, du vanadium, du titane, du fer, de l'indium, du cuivre et de l'aluminium sur la surface de la couche de revêtement d'argent qui est adjacente à au moins une couche de peinture; et au moins une couche de peinture couvrant la couche de revêtement d'argent.

Le brevet EP1113886B décrit quant à lui un procédé pour protéger la couche d'argent d'un miroir contre la corrosion par la mise en contact de la couche d'argent avec une première solution contenant un cation et une seconde solution contenant un anion ou un ion hydroxyle, les deux solutions réagissant ensemble pour former un précipité à la surface de la couche d'argent.

### 3. Objectifs de l'invention

L'invention a notamment pour objectif de fournir un procédé simple et efficace pour protéger un miroir contre la corrosion et le vieillissement.

Un autre objectif de l'invention, dans au moins un de ses modes de réalisation, est de mettre en œuvre un tel procédé pour fournir un miroir qui conserve une bonne résistance mécanique.

L'invention, dans au moins un de ses modes de réalisation, a encore pour objectif de fournir un procédé simple et efficace pour protéger un miroir contre la corrosion et qui respecte l'environnement.

L'invention, dans au moins un de ses modes de réalisation, a encore pour objectif de fournir un miroir dépourvu d'une couche de cuivre.

Un autre objectif de l'invention est de fournir un procédé simple et efficace pour protéger un miroir contre la corrosion et qui permet de s'affranchir d'un revêtement de ladite couche avec une peinture contenant du plomb.

### 4. Exposé de l'invention

L'invention concerne un procédé de fabrication d'un miroir sans couche de cuivre comprenant les étapes suivantes :
a) Se munir d'un substrat en verre,
b) Mettre en contact le substrat en verre avec une solution d'argenture de manière à former une couche d'argent sur le substrat,
c) Réaliser une séquence d'étapes consistant essentiellement en :
   i. Mettre en contact la couche d'argent avec une seule solution de passivation comprenant des ions de bismuth et des ions d'étain,
   ii. Appliquer au moins une couche de peinture sur la couche d'argent,
   iii. Optionnellement rincer et/ou sécher entre chaque étape.

Les inventeurs ont montré qu'un miroir obtenu selon l'invention est protégé contre la corrosion et le vieillissement par le traitement de la couche d'argent avec des ions ou des sels de bismuth. Cet effet est probablement dû à la présence d'une population d'atomes de bismuth à la surface de la couche d'argent.

La couche d'argent est mise en contact avec une seule solution de passivation comprenant des ions de bismuth et des ions d'étain. Cela présente l'avantage d'être pratique et économique puisqu'il ne faut gérer qu'une seule solution et qu'une seule station de traitement est nécessaire, réduisant ainsi le coût de production et d'entretien.

Les inventeurs ont en effet observé que la combinaison d'une passivation par des ions de bismuth avec une passivation par des ions d'étain permettait une protection contre la corrosion et le vieillissement des miroirs ainsi produits accrue par rapport aux miroirs traditionnellement produits. Cet effet est probablement dû à la présence d'une population d'atomes de bismuth et d'étain à la surface de la couche d'argent.

Les inventeurs ont observé que les atomes de bismuth ou d'étain sont présents à la surface de la couche d'argent sous forme d'îlots, c'est-à-dire qu'ils ne forment pas une couche continue distincte à la surface de la couche d'argent. Leur présence peut être détectée, par exemple, par XPS.

De préférence, la solution de passivation contenant de l'étain est fraîche. Lorsqu'on fabrique une solution d'un sel d'étain, on observe qu'après un certain temps, par exemple dans les 48 heures à la température ambiante, certaines réactions peuvent se produire au sein de la solution, en rendant celle-ci légèrement opalescente.

Dans un mode de réalisation préféré de l'invention, la solution de passivation comprend comme source d'ions de bismuth un sel de bismuth (III) dans une solution aqueuse et en particulier du BiCl₃ dans une solution aqueuse acide. Ainsi, des solutions de sels de bismuth telles que des solutions de Bi(NO₃)₃, de Bi(C₆H₅O₇) ou préférentiellement de BiCl₃, peuvent être utilisées de manière très simple et économique. De telles solutions peuvent être utilisées de manière à appliquer (pulvériser) des quantités d'ions ou sels de bismuth (III) en solution comprises entre 0,1 et 240 mg par mètre carré de substrat en verre revêtu d'une couche d'argent. De préférence, des quantités comprises entre 0,1 et 155 mg/m² et de manière encore plus préférée, des quantités comprises entre 1 et 120 mg/m² sont appliquées sur le substrat en verre revêtu d'une couche d'argent. Ainsi, une quantité de 0,001 à 0,2 mg de Bi, de préférence de 0,001 à 0,1 mg de Bi, par mètre carré de substrat en verre revêtu d'une couche d'argent peut être entièrement suffisante pour passiver efficacement le substrat en verre argenté.

De préférence, le pH de la solution de passivation comprenant des ions de bismuth est compris entre 0,5 et 4,0, de préférence entre 1,0 et 3,0. A pH inférieur à 0,5, la couche d'argent peut subir une attaque acide irréversible et à pH supérieur à 4,0, la quantité d'ions ou de sels de bismuth (III) en solution peut ne pas être suffisante, le bismuth se trouvant alors dans la solution de passivation préférentiellement sous forme de précipité. Cette gamme préférée de pH permet donc de former des solutions actives et efficaces pour l'étape de passivation de la couche d'argent.

En outre, des solutions de passivation contenant des ions ou des sels d'étain (II) peuvent également être utilisées de manière très simple et économique. L'application (pulvérisation) d'une quantité aussi faible que 1 mg d'étain en solution par mètre carré de substrat en verre revêtu d'une couche d'argent est tout-à-fait suffisante pour offrir une protection, et on estime que l'application de quantités plus importantes que 1500 mg/m² n'offre pas d'augmentation proportionnelle de la résistance à la corrosion. En fait, l'utilisation de quantités plus importantes peut avoir un effet nuisible en réduisant l'adhérence entre la couche d'argent et la peinture qui est appliquée ultérieurement. Les meilleurs résultats sont obtenus lorsqu'on applique sur le substrat revêtu, des quantités d'ions ou de sels d'étain (II) en solution comprises entre 0,2 mg et 10 mg par mètre carré de substrat en verre revêtu d'une couche d'argent. De préférence, la solution de passivation contenant des ions d'étain est une solution de chlorure d'étain (SnCl₂) ou de sulfate d'étain (SnSO₄). De manière plus préférée, la solution de passivation contenant les ions d'étain est une solution de SnCl₂.

De préférence, le pH de la solution de passivation comprenant les ions d'étain est compris entre 0,5 et 4,0, de préférence entre 1,0 et 3,0.

Dans le cas où la couche d'argent est traitée avec une seule solution de passivation qui comprend les ions de bismuth et les ions d'étain, les concentrations de ces ions sont de préférence respectivement les mêmes que celles mentionnées ci-dessus. De préférence, le pH de ladite solution est compris entre 0,5 et 4,0, de préférence entre 1,0 et 3,0.

Dans d'autres formes de l'invention, un ou plusieurs autre(s) composé(s) peuvent être déposés pendant l'étape de passivation en combinaison avec des ions de bismuth et d'étain sur la couche d'argent déposée une surface du substrat en verre. De tels composés peuvent être choisis parmi le groupe constitué du chrome, du vanadium, du titane, du fer, de l'indium, du cuivre, de l'aluminium, du palladium, du nickel, de l'europium, du platine, du ruthénium, du sodium, du zirconium, de l'yttrium, du rhodium, du zinc, du cérium.

Les miroirs selon l'invention ont de préférence une bonne résistance au vieillissement et à la corrosion, de préférence au moins comparable à la résistance au vieillissement et à la corrosion des miroirs tels que décrits dans le brevet US 6,565,217. De plus, de tels miroirs peuvent avoir une occurrence inférieure et/ou un risque inférieur et/ou être moins sensibles aux facteurs qui pourraient provoquer des taches de corrosion se répandant dans le miroir.

La couche d'argent dans un miroir fabriqué selon l'invention n'est pas revêtue d'une couche de cuivre, comme elle l'est dans les procédés classiques de fabrication de miroirs. Ceci présente des avantages au point de vue économique et environnemental puisque l'étape classique de cuivrage est supprimée, ce qui économise des matières et du temps de fabrication et de retraitement du cuivre. Il est extrêmement surprenant que la mise en contact d'une couche d'argent avec une solution de traitement selon l'invention, suivie de l'application d'une peinture puisse protéger la couche d'argent contre la corrosion et l'abrasion aussi bien qu'une couche de cuivre classique qui est surmontée d'une peinture contenant un pigment à base de plomb.

Selon l'invention, la couche d'argent est recouverte d'au moins une couche protectrice de peinture, formant ainsi des miroirs argentés sur la face arrière. Le miroir est ainsi pourvu d'une protection contre la corrosion et l'abrasion par le traitement conforme à l'invention et la (les) couche(s) de peinture. Avantageusement, on dépose une telle couche de peinture sur la couche d'argent après que celle-ci ait été traitée au moyen d'un silane. La mise en contact de la couche d'argent avec un silane avant peinture peut favoriser l'adhérence de la peinture sur le revêtement métallique traité, ce qui peut participer à la résistance à l'abrasion et à la corrosion du miroir. De préférence, pour des raisons environnementales, la dite peinture est "substantiellement dépourvue de plomb" ou "sans plomb". De manière conventionnelle, les couches d'argent des miroirs étaient protégées par une couche de cuivre. La couche de cuivre était ensuite protégée de l'abrasion et la corrosion par une couche de peinture contenant du plomb. La proportion de plomb dans une telle couche de peinture peut atteindre des taux d'environ 13000 mg/m². Les miroirs selon la présente invention s'affranchissent non seulement du besoin d'une couche de cuivre mais ils permettent aussi l'utilisation de peintures qui sont "substantiellement dépourvues de plomb" ou "sans plomb". Ceci est avantageux puisque le plomb est toxique et le fait de s'en passer a des avantages environnementaux. "Substantiellement dépourvue de plomb" signifie que la proportion de plomb dans la peinture est significativement inférieure à la proportion de plomb contenue classiquement dans les peintures au plomb conventionnellement utilisées pour des miroirs. La proportion de plomb dans une couche de peinture "substantiellement dépourvue de plomb" comme définie, est inférieure à 500 mg/m², de préférence inférieure à 400 mg/m², plus préférentiellement inférieure à 300 mg/m². "Sans plomb" signifie que la proportion de plomb dans la peinture est inférieure à 10 mg/m², de préférence inférieure à 5 mg/m². La présente invention offre l'avantage de pouvoir utiliser une peinture "sans plomb", tout en garantissant une bonne résistance au vieillissement et à la corrosion, de préférence une résistance au moins comparable à la résistance au vieillissement et à la corrosion des miroirs décrits dans le brevet US 6,565,217. Elle peut aussi offrir l'avantage d'utiliser une peinture "substantiellement dépourvue de plomb" d'épaisseur réduite, en ayant toujours une bonne résistance au vieillissement et à la corrosion, de préférence au moins comparable à la résistance au vieillissement et à la corrosion des miroirs tels que décrits dans le brevet US 6,565,217.

La solution de passivation peut également contenir un additif tel que du β-naphtol, qui a pour effet d'augmenter la stabilité des ions dans la solution, en particulier les ions d'étain (lI).

Les inventeurs ont trouvé que l'efficacité du traitement conforme à l'invention est favorisée lorsque, ainsi qu'on le préfère, la solution de traitement a un pH qui n'est pas supérieur à 4. L'acidification de la solution de traitement est obtenue de manière appropriée en ajoutant l'acide correspondant aux sels de bismuth à la solution comprenant les sels de bismuth ou à la solution comprenant un mélange des sels de bismuth et d'étain.

Avantageusement, un ou plusieurs composé(s) peuvent être déposés pendant une étape d'activation sur une surface du substrat en verre sur laquelle la couche d'argent doit être déposée; ceci peut contribuer à la résistance à la corrosion du miroir. De tels composés peuvent être choisis parmi le groupe constitué du palladium, du bismuth, du chrome, de l'or, de l'indium, du nickel, du platine, du rhodium, du ruthénium, du titane, du vanadium et du zinc. Le palladium est préféré.

Durant une étape de sensibilisation, de l'étain est préférentiellement déposé à la surface du substrat en verre sur lequel la couche d'argent doit être déposée; ceci peut sensibiliser le substrat en verre et peut faciliter l'adhérence de la couche d'argent.

De préférence, les composés déposés à la surface du substrat en verre pendant les étapes d'activation et/ou de sensibilisation sont déposés sous forme d'îlots, c'est-à-dire qu'ils ne forment pas une couche continue distincte, par exemple, de palladium, mais que le composé est sous forme d'îlots à la surface du verre.

Dans des méthodes de fabrication de miroirs selon certains aspects de l'invention, les étapes de sensibilisation et d'activation peuvent contribuer à la résistance au vieillissement et/ou à la corrosion des miroirs et/ou à leur durabilité. Selon un mode de réalisation particulier de l'invention, l'étape d'activation est effectuée avant l'étape de sensibilisation ou les étapes d'activation et de sensibilisation sont effectuées simultanément. De préférence l'étape de sensibilisation est effectuée avant l'étape d'activation et l'étape d'activation avant l'étape d'argenture.

De préférence, les solutions mises en contact avec le substrat en verre pendant les étapes de fabrication successives (sensibilisation, activation, argenture, passivation, silane) sont pulvérisées sur le substrat en verre avec optionnellement des étapes de rinçage et/ou de séchage.

Par exemple, pendant la fabrication industrielle de miroirs plats, des feuilles de verre passent au travers de stations successives où une solution de sensibilisation, une solution d'activation et des réactifs d'argenture sont pulvérisés. En pratique, sur une chaîne de production de miroirs, les feuilles de verre passent généralement d'une station à une autre le long d'un convoyeur. Elles sont tout d'abord polies et rincées avant d'être sensibilisées par exemple à l'aide d'une solution de chlorure d'étain pulvérisée sur le verre; elles sont alors rincées de nouveau. Une solution d'activation est alors pulvérisée sur les feuilles de verre, cette solution d'activation peut être par exemple, une solution aqueuse acide de PdCl₂. Les feuilles de verre passent alors à une station de rinçage où de l'eau déminéralisée est pulvérisée et passent ensuite à la station d'argenture où une solution d'argenture traditionnelle est pulvérisée, la solution d'argenture étant la combinaison, juste avant son application sur le verre de deux solutions, une solution comprenant un sel d'argent et un agent de réduction ou une base et l'autre solution comprenant n'importe quel agent de réduction ou une base qui est absent de la solution contenant le sel d'argent. Le débit et la concentration de la solution d'argenture pulvérisée sur le verre sont contrôlés afin de former une couche d'argent contenant entre 650 et 1500 mg/m² d'argent, de préférence dans la gamme de 700-1200 mg/m² d'argent et de manière plus préférée dans la gamme de 700-1000 mg/m² d'argent. Le verre est alors rincé et directement après le rinçage de la couche d'argent, une première solution de passivation acidifiée fraîchement formée par exemple de chlorure d'étain (SnCl₂) est pulvérisée sur les feuilles de verre argentées durant leur progression le long du convoyeur. Après un rinçage à l'eau déminéralisée, une seconde solution de passivation est alors pulvérisée sur la couche d'argent, cette solution de passivation selon l'invention est une solution aqueuse acide contenant des ions de bismuth, formée par exemple de chlorure de Bi (BiCl₃). De préférence, la couche d'argent passivée est ensuite traitée avec une solution contenant un silane et dans ce cas particulier, la couche d'argent est nécessairement rincée entre les étapes de passivation et le traitement avec le silane. Après traitement avec la solution contenant un silane, rinçage et séchage, les miroirs sont couverts d'au moins une couche de peinture. La peinture est alors chauffée (réticulée) ou séchée, par exemple dans un four.

Ainsi, les miroirs selon la présente invention ont de préférence une résistance acceptable ou même améliorée au vieillissement et/ou la corrosion; ceci est défini notamment par le CASS test et/ou le test du brouillard salin encore appelé le Neutral Salt Spray test.

Une indication de la résistance au vieillissement d'un miroir incorporant une couche d'argent peut être donnée en le soumettant à un test comprenant la pulvérisation d'un sel de cuivre et d'acide acétique, connu sous le nom de CASS test, dans lequel le miroir est placé dans une enceinte d'essai à 50°C et est soumis à l'action d'un brouillard formé par pulvérisation d'une solution aqueuse contenant 50 g/l de chlorure de sodium, 0,2 g/l de chlorure cuivreux anhydre avec suffisamment d'acide acétique glacial pour porter le pH de la solution pulvérisée à une valeur comprise entre 3,0 et 3,1. Les détails complets concernant ce test sont donnés dans la norme internationale ISO 9227. Des miroirs peuvent être soumis au CASS test pendant différentes durées, ensuite les propriétés réfléchissantes du miroir vieilli artificiellement peuvent être comparées avec les propriétés réfléchissantes du miroir fraîchement formé. Selon la norme EN 1036-1, un temps d'exposition de 120 heures permet d'avoir une indication sur la résistance d'un miroir au vieillissement. On exécute le CASS test sur des miroirs carrés de 10 cm de côté, et après exposition de 120 heures au brouillard d'acide acétique et de sel de cuivre, chaque miroir est soumis à un examen microscopique. La preuve visible principale de corrosion est l'assombrissement de la couche d'argent et le décollement de la peinture aux bords du miroir. Le niveau de corrosion est noté en cinq points régulièrement espacés sur deux bords opposés du carré et on calcule la moyenne de ces dix mesures. On peut également mesurer la corrosion maximale présente au bord du carré pour obtenir un résultat qui est lui aussi mesuré en *µ*m.

Une seconde indication de la résistance au vieillissement d'un miroir incorporant un film métallique peut être donnée en le soumettant au "test de brouillard salin" qui consiste à soumettre le miroir à l'action, dans une enceinte maintenue à 35°C, d'un brouillard salin formé par pulvérisation d'une solution aqueuse contenant 50 g/l de chlorure de sodium. Selon la norme EN 1036-1, un temps d'exposition de 480 heures au test de brouillard salin permet d'avoir une indication sur la résistance au vieillissement d'un miroir. De nouveau, le miroir est soumis à l'examen microscopique, et la corrosion présente au bord du carré est mesurée pour obtenir un résultat en *µ*m, de la même manière que dans le CASS test.

L'invention s'étend également à l'utilisation d'ions de bismuth en combinaison avec des ions d'étain pour passiver une couche d'argent appliquée sur un substrat en verre lors d'un procédé de fabrication d'un miroir sans couche de cuivre. Selon cette utilisation, les ions de bismuth proviennent d'une solution aqueuse de BiCl₃.

Les avantages dues à cette utilisation sont les mêmes que ceux obtenus pour le procédé de fabrication d'un miroir selon l'invention, ils ne sont pas décrits plus amplement.

Des formes préférées de réalisation de l'invention seront maintenant décrites à titre d'exemples seulement.

### EXEMPLES

### Exemple 1 de référence et exemple comparatif 1

Des miroirs selon l'invention sont fabriqués sur une ligne classique de production de miroirs. Les feuilles de verre passent alors d'une station de traitement à une autre à l'aide d'un convoyeur. Les feuilles de verre sont polies et sensibilisées au moyen d'une solution de chlorure stanneux (SnCl₂), de manière habituelle. Après rinçage, les feuilles sont ensuite activées au moyen d'une solution de chlorure de palladium (PdCl₂). Les feuilles de verre sont alors rincées par vaporisation d'eau déminéralisée. On pulvérise ensuite sur les feuilles une solution d'argenture classique contenant un sel d'argent et un agent réducteur, le débit de pulvérisation étant tel qu'il se forme sur chaque feuille une couche contenant environ 750-850 mg/m² d'argent. Le verre argenté est ensuite rincé. Une solution de passivation aqueuse acidifiée est vaporisée sur la feuille de verre de manière à pulvériser sur celle-ci 106 mg/m² de SnCl₂, afin que soit déposé 0,8 mg SnCl₂/m² sur le substrat en verre revêtu de la couche d'argent. La solution de chlorure stanneux utilisée est fraîchement préparée. Le pH de la solution pulvérisée est d'environ 2,5. Après un tel traitement, le verre est rincé et traité avec une solution de passivation aqueuse acidifiée contenant du BiCl₃ de manière à pulvériser sur la feuille de verre 232 mg/m² de BiCl₃, afin que soit déposée sur le substrat en verre revêtu de la couche d'argent une quantité de 0,08 mg/m² de Bi. Le pH de la solution pulvérisée est d'environ 1,5. Le verre est ensuite rincé, séché et recouvert de deux couches de peintures substantiellement dépourvues de plomb. Ainsi, une première couche de la peinture LLE (commercialisée par Fenzi) et une seconde couche de la peinture AW (commercialisée par Fenzi) sont déposées, les deux couches ayant chacune une épaisseur d'environ 25 *µ*m.

Un exemple comparatif est produit comme décrit précédemment excepté que l'étape de pulvérisation de la solution de passivation comprenant les ions de bismuth (BiCl₃) n'est pas effectuée. L'exemple comparatif 1 correspond à un miroir sans cuivre de l'art antérieur.

Les miroirs fabriqués de cette manière sont soumis au test de vieillissement accéléré, le CASS test. Les résultats des tests sur les miroirs de l'exemple 1 et l'exemple comparatif 1 sont repris dans la table 1. Comme le montre la table 1, le miroir obtenu selon l'invention présente une valeur de CASS test inférieure à celle obtenue pour l'exemple comparatif 1. Ainsi, la passivation de la couche d'argent avec une solution comprenant des ions de bismuth améliore la résistance à la corrosion par rapport au miroir de l'art antérieur.

| **TABLE 1 Résultats du CASS Test** | **Exemple 1** | **Exemple comparatif 1** |
|---|---|---|
| Corrosion de bord moyenne (en *µ*m) | 74 | 81 |
| Corrosion de bord maximale (en *µ*m) | 111 | 117 |

### Exemple 2 selon l'invention

Le miroir selon l'exemple 2 est fabriqué de la même manière que celle décrite dans l'exemple 1 à l'exception de l'étape de passivation de la couche d'argent. En effet, la couche d'argent est traitée avec une seule solution de passivation comprenant les ions de bismuth et les ions d'étain. Ainsi, une solution de passivation aqueuse acidifiée, à un pH d'environ 2,5 est vaporisée sur la feuille de verre de manière à pulvériser sur la feuille de verre 103 mg/m² de BiCl₃ et 106 mg/m² de SnCl₂, afin que soient déposées sur le substrat en verre revêtu de la couche d'argent une quantité de 0,01 mg/m² de Bi et une quantité de 1 mg/m² de Sn. Le verre est ensuite rincé, séché et recouvert de deux couches de peintures substantiellement dépourvues de plomb. Ainsi, une première couche de la peinture LLE (commercialisée par Fenzi) et une seconde couche de la peinture AW (commercialisée par Fenzi) sont déposées, les deux couches ayant chacune une épaisseur d'environ 25 *µ*m.

Les miroirs fabriqués de cette manière sont soumis au test de vieillissement accéléré, le CASS test. Les résultats des tests sur les miroirs de l'exemple 2 et l'exemple comparatif 1 sont repris dans la table 2. Comme le montre la table 2, le miroir obtenu selon l'invention présente une valeur de CASS test inférieure à celle obtenue pour l'exemple comparatif 1. Ainsi, la passivation de la couche d'argent avec une solution comprenant à la fois des ions de bismuth et des ions d'étain améliore également la résistance à la corrosion par rapport au miroir de l'art antérieur.

| **TABLE 2 Résultats du CASS Test** | **Exemple 2** | **Exemple comparatif 1** |
|---|---|---|
| Corrosion de bord moyenne (en *µ*m) | 71 | 81 |
| Corrosion de bord maximale (en *µ*m) | 105 | 117 |

### Exemples 3 et 4 de référence, exemple 5 selon l'invention et exemple comparatif 2

Des miroirs selon les exemples 3 à 5 sont fabriqués de la même manière que celle décrite dans les exemples 1 et 2, à l'exception de l'étape de passivation de la couche d'argent et le type de peinture utilisé pour recouvrir la couche d'argent.

Exemple 3: La feuille de verre recouverte d'une couche d'argent est d'abord traitée avec une solution de passivation aqueuse acidifiée contenant du chlorure stanneux. Ainsi une solution de passivation aqueuse est vaporisée sur la feuille de verre de manière à pulvériser sur celle-ci 106 mg/m² de SnCl₂. La solution de chlorure stanneux utilisée est fraîchement préparée. Le pH de la solution pulvérisée est d'environ 2,5. Après un tel traitement, le verre est rincé et traité avec une solution de passivation aqueuse acidifiée contenant du BiCl₃ de manière à pulvériser 68 mg/m² de BiCl₃. Le pH de la solution pulvérisée est d'environ 3. Le verre est ensuite rincé, séché et recouvert d'une couche d'une peinture sans plomb d'une épaisseur d'environ 47 *µ*m.

Exemple comparatif 2: un miroir a été produit comme décrit précédemment à l'exception que l'étape de pulvérisation de la solution de passivation comprenant les ions de bismuth (BiCl₃) a été omise. L'exemple comparatif 2 correspond à un miroir sans cuivre de l'art antérieur.

Exemple 4: La feuille de verre recouverte d'une couche d'argent est d'abord traitée avec une solution de passivation aqueuse acidifiée contenant du chlorure stanneux. Ainsi une solution de passivation aqueuse est vaporisée sur la feuille de verre de manière à pulvériser sur celle-ci 106 mg/m² de SnCl₂. La solution de chlorure stanneux utilisée est fraîchement préparée. Le pH de la solution pulvérisée est d'environ 2,5. Après un tel traitement, le verre est rincé et traité avec une solution de passivation aqueuse acidifiée contenant du BiCl₃ de manière à pulvériser 108 mg/m² de BiCl₃ sur la couche d'argent. Le pH de la solution pulvérisée est d'environ 2.

Exemple 5 selon l'invention est produit en pulvérisant sur la couche d'argent une seule solution de passivation comprenant les ions de bismuth et les ions d'étain. Ainsi, une solution de passivation aqueuse acidifiée, à un pH d'environ 2,5, contenant du chlorure stanneux (SnCl₂) et du chlorure de bismuth (BiCl₃) est vaporisée sur la feuille de verre de manière à pulvériser sur celle-ci 68 mg/m² de SnCl₂ et 68 mg/m² de BiCl₃. Le verre est ensuite rincé, séché et recouvert d'une couche de peinture sans plomb d'une épaisseur d'environ 47*µ*m.

Les miroirs fabriqués de cette manière sont soumis au test de vieillissement accéléré, le CASS test. Les résultats des tests sur les miroirs des exemples 3 à 5 et l'exemple comparatif 2 sont repris dans la table 3. Comme le montre la table 3, les miroirs obtenus selon l'invention présentent une valeur de CASS test inférieure à celle obtenue pour l'exemple comparatif 2. Ainsi, la passivation de la couche d'argent avec une solution comprenant à la fois les ions de bismuth et les ions d'étain améliore la résistance à la corrosion par rapport au miroir de l'art antérieur. De plus de manière surprenante, les inventeurs ont montré que l'effet dû à la présence d'ions de bismuth à la surface de la couche d'argent est plus important lorsqu'une peinture sans plomb est utilisée pour protéger la couche d'argent contre la corrosion et le vieillissement. La table 3 montre également que le traitement de la couche d'argent avec une solution de passivation comprenant les ions de bismuth et une deuxième solution de passivation comprenant les ions d'étain ou encore une seule solution de passivation comprenant les ions de bismuth et d'étain améliore de façon significative la résistance à la corrosion et au vieillissement des miroirs ainsi fabriqués.

| **Table 3** | **Exemple 3** | **Exemple 4** | **Exemple 5** | **Exemple comp 2** |
|---|---|---|---|---|
| **Résultats du CASS Test** | | | | |
| Corrosion de bord moyenne (en *µ*m) | 328 | 330 | 283 | 718 |

## Revendications

1. Procédé de fabrication d'un miroir sans couche de cuivre comprenant les étapes suivantes :
a) Se munir d'un substrat en verre,
b) Mettre en contact le substrat en verre avec une solution d'argenture de manière à former une couche d'argent sur le substrat,
c) Réaliser une séquence d'étapes consistant essentiellement en :
i. Mettre en contact la couche d'argent avec une seule solution de passivation comprenant des ions de bismuth et des ions d'étain,
ii. Appliquer au moins une couche de peinture sur la couche d'argent
iii. Optionnellement rincer et/ou sécher entre chaque étape.

2. Procédé selon la revendication précédente, **caractérisé en ce que** la solution de passivation a un pH compris entre 0,5 et 4.

3. Procédé selon l'une quelconque des revendications précédentes, **caractérisé en ce que** la quantité d'ions de bismuth appliquée sur ledit substrat en verre revêtu d'une couche d'argent est comprise entre 0,1 et 240 mg/m².

4. Procédé selon l'une quelconque des revendications précédentes, **caractérisé en ce que** la quantité d'ions de bismuth présente sur ledit substrat en verre revêtu d'une couche d'argent est comprise entre 0,001 et 1 mg/m².

5. Procédé selon l'une quelconque des revendications précédentes **caractérisé en ce que** la quantité d'ions d'étain appliquée sur ledit substrat en verre revêtu d'une couche d'argent est comprise entre 1 et 1500 mg/m².

6. Procédé selon l'une quelconque des revendications 1 à 4, **caractérisé en ce que** la quantité d'ions d'étain présente sur ledit substrat en verre revêtu d'une couche d'argent est comprise entre 0,2 et 10 mg/m².

7. Procédé selon l'une quelconque des revendications précédentes, **caractérisé en ce que** la solution de passivation comprend comme source des ions de bismuth une solution aqueuse de chlorure de bismuth.

8. Procédé selon l'une quelconque des revendications précédentes, **caractérisé en ce que** la solution de passivation comprend comme source des ions d'étain une solution aqueuse de chlorure d'étain.

9. Procédé selon l'une quelconque des revendications précédentes, **caractérisé en ce que** l'étape b) est précédée d'une étape de sensibilisation et une étape d'activation.

10. Procédé selon la revendication précédente, **caractérisé en ce que** l'étape d'activation comprend une étape de mise en contact de la surface du substrat avec une solution comprenant au moins un ion choisi parmi Bi, Cr, Au, In, Ni, Pd, Pt, Rh, Ru, Ti, V et Zn.

11. Procédé selon l'une des revendications précédentes, **caractérisé en ce que** l'étape d'application d'au moins une peinture est précédée d'une étape de rinçage suivie d'un traitement avec une solution comprenant du silane.

12. Utilisation d'ions de bismuth en combinaison avec d'ions d'étain pour passiver une couche d'argent appliquée sur un substrat en verre lors d'un procédé de fabrication d'un miroir sans couche de cuivre.

## Patentansprüche

1. Verfahren zur Herstellung eines Spiegels ohne Kupferschicht, das Folgendes umfasst:
a) Bereitstellen eines Glassubstrats,
b) Inkontaktbringen des Glassubstrats mit einer Versilberungslösung zur Bildung einer Silberschicht auf dem Substrat,
c) Durchführen einer Abfolge von Schritten, die im Wesentlichen aus Folgendem besteht:
i. Inkontaktbringen der Silberschicht mit einer einzigen Passivierungslösung, die Bismutionen und Zinnionen umfasst,
ii. Aufbringen mindestens einer Anstrichmittelschicht auf die Silberschicht,
iii. Gegebenenfalls Spülen und/oder Trocknen zwischen jedem Schritt.

2. Verfahren nach dem vorhergehenden Anspruch, **dadurch gekennzeichnet, dass** die Passivierungslösung einen pH-Wert zwischen 0,5 und 4 aufweist.

3. Verfahren nach einem der vorhergehenden Ansprüche, **dadurch gekennzeichnet, dass** die auf das mit einer Silberschicht beschichtete Glassubstrat aufgebrachte Menge an Bismutionen zwischen 0,1 und 240 mg/m² liegt.

4. Verfahren nach einem der vorhergehenden Ansprüche, **dadurch gekennzeichnet, dass** die auf das mit einer Silberschicht beschichtete Glassubstrat aufgebrachte Menge an Bismutionen zwischen 0,001 und 1 mg/m² liegt.

5. Verfahren nach einem der vorhergehenden Ansprüche, **dadurch gekennzeichnet, dass** die auf das mit einer Silberschicht beschichtete Glassubstrat aufgebrachte Menge an Zinnionen zwischen 1 und 1500 mg/m² liegt.

6. Verfahren nach einem der Ansprüche 1 bis 4, **dadurch gekennzeichnet, dass** die auf das mit einer Silberschicht beschichtete Glassubstrat aufgebrachte Menge an Zinnionen zwischen 0,2 und 10 mg/m² liegt.

7. Verfahren nach einem der vorhergehenden Ansprüche, **dadurch gekennzeichnet, dass** die Passivierungslösung als Quelle von Bismutionen eine wässrige Lösung von Bismutchlorid umfasst.

8. Verfahren nach einem der vorhergehenden Ansprüche, **dadurch gekennzeichnet, dass** die Passivierungslösung als Quelle von Zinnionen eine wässrige Lösung von Zinnchlorid umfasst.

9. Verfahren nach einem der vorhergehenden Ansprüche, **dadurch gekennzeichnet, dass** dem Schritt b) ein Sensibilisierungsschritt und ein Aktivierungsschritt vorausgeht.

10. Verfahren nach dem vorhergehenden Anspruch, **dadurch gekennzeichnet, dass** der Aktivierungsschritt einen Schritt des Inkontaktbringens der Oberfläche des Substrats mit einer Lösung, die mindestens ein aus Bi, Cr, Au, In, Ni, Pd, Pt, Rh, Ru, Ti, V und Zn ausgewähltes Ion umfasst, umfasst.

11. Verfahren nach einem der vorhergehenden Ansprüche, **dadurch gekennzeichnet, dass** dem Schritt des Aufbringens mindestens eines Anstrichmittels ein Spülschritt gefolgt von einer Behandlung mit einer Lösung, die Silan umfasst, vorausgeht.

12. Verwendung von Bismutionen in Kombination mit Zinnionen zur Passivierung einer auf ein Glassubstrat aufgebrachten Silberschicht bei einem Verfahren zur Herstellung eines Spiegels ohne Kupferschicht.

## Claims

1. Process for manufacturing a mirror without a copper layer, comprising the following steps:
a) providing a glass substrate,
b) placing the glass substrate in contact with a silvering solution so as to form a silver layer on the substrate,
c) performing a sequence of steps consisting essentially in:
i. placing the silver layer in contact with a single passivation solution comprising bismuth ions and tin ions,
ii. applying at least one coat of paint to the silver layer,
iii. optionally rinsing and/or drying between each step.

2. Process according to the preceding claim, **characterized in that** the passivation solution has a pH of between 0.5 and 4.

3. Process according to either of the preceding claims, **characterized in that** the amount of bismuth ions applied to said glass substrate coated with a silver layer is between 0.1 and 240 mg/m².

4. Process according to any one of the preceding claims, **characterized in that** the amount of bismuth ions present on said glass substrate coated with a silver layer is between 0.001 and 1 mg/m².

5. Process according to any one of the preceding claims, **characterized in that** the amount of tin ions applied to said glass substrate coated with a silver layer is between 1 and 1500 mg/m².

6. Process according to any one of Claims 1 to 4, **characterized in that** the amount of tin ions present on said glass substrate coated with a silver layer is between 0.2 and 10 mg/m².

7. Process according to any one of the preceding claims, **characterized in that** the passivation solution comprises, as source of bismuth ions, an aqueous bismuth chloride solution.

8. Process according to any one of the preceding claims, **characterized in that** the passivation solution comprises, as source of tin ions, an aqueous tin chloride solution.

9. Process according to any one of the preceding claims, **characterized in that** step b) is preceded by a sensitization step and an activation step.

10. Process according to the preceding claim, **characterized in that** the activation step comprises a step of placing the surface of the substrate in contact with a solution comprising at least one ion chosen from Bi, Cr, Au, In, Ni, Pd, Pt, Rh, Ru, Ti, V and Zn.

11. Process according to one of the preceding claims, **characterized in that** the step of applying at least one paint is preceded by a rinsing step followed by treatment with a solution comprising silane.

12. Use of bismuth ions in combination with tin ions for passivating a silver layer applied to a glass substrate during a process for manufacturing a mirror without a copper layer.
